Europäisches Patentamt

European Patent Office

Office européen des brevets

(19)

(11) Publication number: **0 305 536**

**A1**

(12) **EUROPEAN PATENT APPLICATION**

published in accordance with Art. 158(3) EPC

(21) Application number: **88902218.2**

(22) Date of filing: **26.02.88**

Data of the international application taken as a basis:

(86) International application number:
**PCT/JP88/00213**

(87) International publication number:
**WO88/06611 (07.09.88 88/20)**

(51) Int. Cl.³: **C 08 L 29/12**
**G 01 N 33/545**

(30) Priority: **27.02.87 JP 42807/87**
**01.02.88 JP 19581/88**

(43) Date of publication of application:
**08.03.89 Bulletin 89/10**

(84) Designated Contracting States:
**DE FR GB**

(71) Applicant: **NIPPON KAYAKU KABUSHIKI KAISHA**
**Tokyo Fujimi Bldg. 11-2 Fujimi 1-chome Chiyoda-ku**
**Tokyo 102(JP)**

(72) Inventor: **TAJIMA, Shigeru**
**675-11, Fujioka**
**Fujioka-shi Gunma-ken 375(JP)**

(72) Inventor: **SUZUKI, Takayuki**
**10-26, Soujamachi 2-chome**
**Maebashi-shi Gunma-ken 371(JP)**

(74) Representative: **Türk, Gille, Hrabal**
**Bruckner Strasse 20**
**D-4000 Düsseldorf 13(DE)**

(54) **COLORED LATEX AND LATEX FOR DIAGNOSTIC REAGENT.**

(57) A colored latex obtained by dyeing polyacrolein with a dye, a latex for diagnostic reagent containing said latex, and a latex for diagnostic reagent to be used in the microtiter method comprising said latex are disclosed.

EP 0 305 536 A1

## DESCRIPTION

## COLORED LATEX AND LATEX FOR USE AS
## DIAGNOSTIC REAGENT

### TECHNICAL FIELD:

The present invention relates to a colored latex, and to a latex for use as diagnostic reagent for disease utilizing an antigen-antibody reaction or the like.

### BACKGROUND ART:

In conventional diagnosis of disease, a variety of tests on the serum or urine taken from a patient have been carried out to aid a clinician in diagnosis. Among the tests, for instance, an immunological test comprises testing whether or not an antigen or antibody is present in the body fluid, such as blood and urine, of a patient or the like, by utilizing an antigen-antibody reaction. The reaction is based on the property of an antibody such that it reacts only with a specified antigen, namely, the specificity of the antibody, therefore, it is possible to detects a trace of an antigen or antibody by employing the antigen-antibody reaction.

In the test based on the antigen-antibody

- 2 -

0305536

reaction, an antibody for an antigen to be detected which is present in a body fluid such as serum, urine, etc. is mixed into the body fluid, or, alternatively, an antigen for an antibody to be detected which is present in the body fluid is mixed into the body fluid, thereby causing the antigen-antibody reaction and detecting the formation of an antigen-antibody complex. In the case of direct detection of the antigen-antibody complex, the antigen or the antibody must be present in a large amount. If the amount of the antigen or antibody is small, an insoluble precipitate (antigen-antibody complex) is not formed, and the intended detection cannot be achieved. To enhance the detection sensitivity, a variety of techniques have been proposed.

The method most commonly used at present is a method in which an antigen or antibody is caused to sensitize (be adsorbed on) a large carrier such as red blood cell, a synthetic latex mainly composed of polystyrene, thereby facilitating the detection of the complex derived through an antigen-antibody reaction. The method has high sensitivity in detection, and is widely used.

In a hemagglutination method called the (reversed) passive hemagglutination method in which red blood cells are used, a protein antigen or antibody is adsorbed on the surfaces of red blood cells treated with tannic acid or the like, and the sensitized red blood cells thus obtained are used for measurement of an antibody or antigen present in a body fluid. This method, generally carried out by the microtiter technique using a microplate, has a detection sensitvity as high as 1 to 10 ng/mℓ in terms of the concentration of protein in the body fluid, and is used for various tests.

It is said, however, the red blood cells, originating in living bodies, have differences depending on the species of the living bodies, and even the same species have individual differences and seasonal differnces. Therefore, it is said that red blood cells are liable to vary from lot to lot and the reproducibility thereof is low. Besides, the red blood cells are poor in preservation stability because they are constituents of living bodies.

To overcome the disadvantage arising from the use of red blood cells, attempts have been made to apply synthetic latex to the microtiter technique. There has thus been an attempt to shorten the time required until interpretation and a high interpretation accuracy comparable to those of the hemagglutination method in which red blood cells are used, by increasing the particle diameter and specific gravity of the synthetic latex.

Of various synthetic latexes, polyacrolein latex has a higher specific gravity and is capable of control of particle size. The polyacrolein latex has the unique property of being capable of sensitization with an antigen or antibody through covalent bonds by aldehyde groups present on the particle surfaces thereof. The polyacrolein latex particles, however, have a disadvantage in that they are liable to cause nonspecific agglutination reactions (namely, agglutination reactions other than the antigen-antibody reaction which is the major reaction in an immunological test, for instance, agglutination due to adsorption) because of the excessive activity of the particle surfaces. The nonspecific agglutination reactions, though not being the objective antigen-antibody reaction, results in agglutination, which may induce an error in diagnosis. The polyacrolein latex has another disadvantage in that the sensitized diagnostic latex is poor in preservation stability.

DISCLOSURE OF THE INVENTION:

To solve the above-mentioned problems, the present inventors have made studies of the surface treatments of polyacrolein latex particles and, as a result of the studies, have attained the present invention.

Namely, the present inventors have made intensive studies about an increase and decrease of the nonspecific agglutination reactions, by treating polyacrolein with various compounds in a water bath. As a result of the studies, it has been found that when latex particles are surface treated with an amino group-containing compound or hardly water-soluble compound having a high melting (or decomposition) point, it is possible to inhibit the nonspecific agglutination reactions of the latex particles sensitized with an antigen or antibody. For instance, when bisphenol A was dispersed as a hardly water-soluble compound in water and the latex particles were surface treated in the thus obtained dispersion, it was possible to inhibit the nonspecific agglutination reactions. However, the effect obtained was not sufficient, leaving a problem on a practical use.

Based on the above finding, the present inventors have made earnest investigations on various compounds and, as a result of the investigations, have found that the compounds generically called "dyes" are suitable for solving the problems associated with the polyacrolein latex, from the viewpoints of effect of surface treatment, preservation, stability, easiness of interpretation, etc. Based on the findings the present

0305536

invention has been attained.

Accordingly, the present invention relates to

1. a colored latex prepared by dyeing polyacrolein with a dye;

2. a latex for use as a diagnostic reagent, comprising a colored latex prepared by dyeing polyacrolein with a dye; and

3. a latex for use as a diagnostic reagent in the microtiter technique, comprising a colored latex prepared by dyeing polyacrolein with a dye.

According to the present invention, it is possible to obtain easily a colored latex useful as a diagnostic reagent. The diagnostic reagent obtained by using the colored latex of the invention is low in possibility of nonspecific agglutination reactions and is excellent in preservation stability. Besides, the use of the dye has a subsidiary effect of facilitating interpretation in the microtiter technique and, therefore, shortening the time required until interpretation.

The easiness of occurence of the nonspecific agglutination reactions in the case of using the diagnostic reagent based on naked polyacrolein latex not dyed with a dye is considered to be due to functional groups present on the surfaces of the latex particles, hydrophilic-hydrophobic balance, or static charges on the surfaces, etc. It is considered that the inhibition of the nonspecific agglutination reactions is the result of changes in physical or chemical properties of the particle surfaces caused by coating of the particle surfaces with the dye upon dyeing. The reason for the effect of improving the preservation stability of the diagnostic reagent is considered as follows. The polyacrolein latex, per se, has a complicated structure and instability. Namely, when acrolein monomer is subjected to polymerization, not only the vinyl polymerization takes place but the carbonyl double bond conjugated with the carbon-carbon double bond participates in the polymerization, as a result, the polyacrolein formed has a complicated structure and unstable bonds. However, upon the surface treatment of the polyacrolein latex particles by dyeing, the latex particles seem to be stabilized by linkage to the dye molecules, and/or strong affinity to the dye molecules, etc.

As the method for dyeing a synthetic latex by using a dye, a variety of methods have been adopted, for instance, a method of mixing the dye into a mixture of monomers at the time of producing the latex, a method of using an organic solvent to swell the latex particles together with the dye, thereby coloring the latex particles, a method of kneading the dye into the latex, etc. However, there has not been adopted a method of coating the material to be

0305536

dyed in water, as in the case of dyeing a woven fabric, based on the concept of surface treatment. A dye is bonded to the polyacrolein latex particles, starting from the surfaces of the latex particles, by the affinity of the dye molecule and the polyacrolein particle for each other, so that the dye has a high surface-treating effect and is able to show a sufficient effect even when used in a small amount. Therefore, though it is naturally possible to dye the polyacrolein latex particles to the inside portions of the particles, simple coating of the particle surfaces with the dye also show a sufficient surface-treating effect and it is possible, by such simple coating, to inhibit the nonspecific agglutination reactions. According to the above-mentioned method of mixing the dye with a monomer at the time of producing the latex, the latex particles are uniformly dyed to the inside thereof, so that the surface-treating effect is slight.

The undyed polyacrolein latex for use in the present invention, per se, is well known and is capable of being obtained by various production methods. For instance, the polyacrolein latex is obtainable by employing emulsion polymerization with radical polymerization, alkali-induced polymerization, photopolymerization or radiation polymerization, the particle diameter of the polyacrolein latex being preferably 0.1 to 5 μm.

The dye for use in the present invention may be any dye which has a dyeing ability. Thus, most of the dyes classified as basic dyes, acid dyes, direct dyes, disperse dyes, reactive dyes or the like are usable. Among these dyes, amino group-containing basic dyes and acid dyes, and hydrophobic fluorescent dyes and disperse dyes, particularly, have a high effect on inhibition of the nonspecific agglutination reactions and a high effect on preservation stability.

It is possible to obtain the colored latex by a dyeing method according to the kind of the dye used. By selecting appropriate sensitizing conditions, it is possible to obtain a diagnostic reagent applicable to the microtiter technique or the like. Besides, by use of a polyacrolein latex controlled to a relatively small particle diameter, it is possible to apply the resultant diagnostic reagent to an optical measurement method or a plate agglutination method.

The names of examples of the dye, for example, blue dyes, for use in the present invention will now be set forth below.

Kayarus Supra Blue BRL

Kayaku Direct Blue 2BA

Kyacryl Blue GRL Kayanol Blue NR

Kyanoi Milling Blue BW

Kayakalan Blue Black RL

Kayaction Blue P-N3G

Kayaset Blue A-2R

Kayalon Polyester Blue 2R-SF (the above are products by Nippon Kayaku Kabushiki Kaisha)

Crystal Violet  (this is a product by Wako Junyaku Kabushiki Kaisha.)

The dye for use in the present invention,
however, is not limited to those in the blue group, and it
is natural that other dyes, for instance, Kayalon
Polyester Light Yellow 4G-S Paste (yellow), Kayacryl
Red GRL (red) (these are products by Nippon Kayaku
Kabushiki Kaisha), Fuchsin (red), Malachite Green (green)
(these are products by Wako Junyaku Kabushiki Kaisha),
etc. can be used to achieve extremely good dyeing.  It is
also possible to dye in an intermediate color by mixing
the dyes.

The dyeing of the polyacrolein latex is carried
out by applying  similar conditions for dyeing
a woven fabric.  The dye is preferably used in solution or
dispersion in water, in a concentration of 0.001 to 5%,
whereas  the solid content of the polyacrolein latex as the material to be dyed is preferably 0.1 to 20%. The dyeing temperature is in the range from normal temperture to 100°C, and the dyeing is finished in a period ranging from 30 minutes to 5 hours. Addition of sodium sulfate, acetic acid, sodium carbonate or the like may be carried out at the time of dyeing, for dyeing in a deep color. Thereafter, the latex is cleaned by filtration, centrifugation, dialysis or the like, whereby a colored latex free of elution of the dye can be obtained.

The polyacrolein solid content in the colored latex according to the present invention is not particularly limited, but it is preferably 0.01 to 40% by weight. After the sensitization with an antigen or antibody, the polyacrolein solid content in the latex is preferably 0.01 to 2% by weight. The dispersant of the latex may be, for example, water or an organic solvent such as an alcohol and acetone. The dispersant used after the sensitization is preferably water. The sensitization of the colored polyacrolein latex can be carried out by a general sensitizing method, and the latex can be easily sensitized with an antibody, an antigen or the like. Namely, the colored latex can be sensitized by bringing it into contact with the antibody, antigen or the like for sensitization in physiological phosphate-buffered saline or the like. By removing the free antibody, antigen or the like and suspending the sensitized latex in physiological phosphate-buffered saline or the like, it is possible to obtain a diagnostic reagent usable in the microtiter technique or the like. When the diagnostic reagent thus obtained was used to carry out an immunological test based on the microtiter technique, the agglutination setting pattern was easy to see, whether positive or negative, and it was possible to make interpretation withour need for skill.

BEST MODE FOR CARRYING OUT THE INVENTION:

The present invention will now be explained in detail below while referring to Examples and Test Examples.

EXAMPLE 1:

To 100 g of a polyacrolein latex (particle diameter 1.0 μm, a product of alkali-induced polymerization) (solid content: 10%), was added 0.2 g of Kayacryi Navy Blue BL (a product of Nippon Kayaku Kabushiki Kaisha) in 100 mℓ of water, and the resultant mixture was stirred for 1 hour on a water bath at 95°C.

Then, 1 mℓ of acetic acid was added, followed by further stirring for 2 hours. After cooling, the supernatent was replaced three times by a 2000 rpm x 5 min centrifugation, and the resultant mixture was dispersed in 200 mℓ of water containing 1 mℓ of acetic acid, and the dispersion was stirred for 30 min on a 95°C water bath in the same manner as for dyeing. After cooling, the resultant mixture was cleaned five times by centrifugation to yield a polyacrolein latex colored in dark blue.

EXAMPLE 2:

To 100 g of a polyacrolein latex (particle diameter 1.5 μm, a product of alkali-induced polymerization followed by radical polymerization) (solid content : 10 %), was added 0.5 g of Kayalon Polyester Light Scarlet G-SP Paste (a product by Nippon Kayaku Kabushiki Kaisha) dissolved in 100 mℓ of water, and the resultant mixture was stirred for 2 hours on a water bath at 95°C. After cooling, the supernatent was replaced five times by a 2000 rpm x 5 min centrifugation to obtain a polyacrolein latex colored in scarlet.

EXAMPLE 3:

Dyeing was carried out in the same manner as in Example 1 by using 0.05 g of Kayacryl Blue GRL (a product by Nippon Kayaku Kabushiki Kaisha) and 0.1 g of Kayacryl Brilliant Yellow 3RL (a product by the same company) per 100 g of the latex used in Example 2, to obtain a polyacrolein latex colored in green.

EXAMPLE 4:

Dyeing was carried out in the same manner as in Example 1 by using 0.2 g of Kayanol Milling Red BW (a product by Nippon Kayaku Kabushiki Kaisha) per 100 g of the latex used in Example 1, to obtain a polyacrolein latex colored in deep red.

EXAMPLE 5:

Dyeing was carried out in the same manner as in Example 1 by using 0.1 g of Crystal Violet (a product by Wako Junyaku Kabushiki Kaisha) per 100 g of the latex used in Example 2, to obtain a polyacrolein latex colored in bluish purple.

COMPARATIVE EXAMPLES 1 AND 2:

The polyacrolein latexes used respectively in Examples 1 and 2, not yet dyed, were prepared.

COMPARATIVE EXAMPLE 3:

A surface treatment was carried out in the same manner as in Example 2 by using 0.2 g of bisphenol A per 100 g of the latex used in Example 2, to obtain a white latex.

TEST EXAMPLE:

The colored latexes obtained in Examples 1 to 5 and the latexes of Comparative Examples 1 to 3 were subjected to sensitization to obtain reagents for an immunological test based on the microtiter technique, and the immunological test was performed.

The milliliters of a 0.05 M physiological phosphate-buffered saline (PBS) containing a polyacrolein latex dispersed therein in a solid content of 0.5%, 50 μℓ of anti-human α-fetoprotein serum ( goat IgG fraction, with a protein concentration of 2.5 mg/mℓ), and 10 mg of PBS with 0.5% bovine serum albumin (BSA) dissolved therein were mixed with each other, and the resultant mixture was slowly shaken at 37°C for 1 hour. Then the mixture was subjected to centrifugation (1500 rpm x 5 min), the precipitants were washed three times with 10 mℓ of PBS containing 0.1% PSA therein, and the washed matter was finally suspended in 10 mℓ of PBS containing 0.1% BSA therein, to obtain a sensitized latex.

Separately, each well of a 96-holed U-type microplate (a product by K.K. Toyoshima Seisakusho) was charged with 25 μℓ of PBS containing 0.1% BSA therein, then 100 ng/mℓ of an AFP standard (a product by K.K. Nippon Bio Test Kenkyusho) and 25 μℓ of normal human serum were added to each well, and two-fold continuous dilution was performed by using a dilutor. To each well, was added 25 μℓ

of the sensitized latex, and after shaking by a mixer, the plate were left to stand at normal temperature. After 2 hours, the agglutination setting pattern in each well was read. Besides, the period of time required for stabilization of the agglutination setting pattern was also measured. Further, the sensitized latexes were preserved at 4°C for 3 months to compare their stability. The results are shown in Table 1. The numerical value of standard in Table 1 represents the degree of dilution giving a positive result immediately before the interpretation result turned negative. The numerical value for normal person is the rate of positive results at a degree of dilution of at least 16 times among 100 sample serum, and represents the incidence of nonspecific agglutination reaction. The interpreting time is represented by the period of time required until interpretation.

0305536

## Table 1

| Latex served to sensitization | Immediately after production of sensitized latex | | Interpreting time (hr:min) | Standard after 3 months |
|---|---|---|---|---|
| | Normal | Normal person | | |
| Ex. 1 | 64 | 2 | 1 : 30 | 64 |
| " 2 | 128 | 1 | 1 : 10 | 128 |
| " 3 | 128 | 2 | 1 : 10 | 128 |
| " 4 | 64 | 2 | 1 : 30 | 64 |
| " 5 | 128 | 2 | 1 : 10 | 128 |
| Comp. Ex. 1 | 64 | 30 | 1 : 50 | 16 |
| " 2 | 128 | 24 | 1 : 25 | 32 |
| " 3 | 128 | 5 | 1 : 50 | 128 |

Standard:   AFP standard   100 ng/mℓ

INDUSTRIAL APPLICABILITY:

As has been described above, according to the present invention it is possible to surface-treat polyacrolein latex particles by dyeing the polyacrolein latex with a dye. As a result, it is possible to obtain a diagnostic reagent which, when used for an immunological test based on the microtiter technique or the like, is low in possibility of nonspecific agglutination reactions, shows good stability and enables easy interpretation.

## CLAIMS

1.      A colored latex prepared by dyeing polyacrolein with a dye.

2.      A latex for use as a diagnostic reagent, comprising a colored latex prepared by dyeing polyacrolein with a dye.

3.      A latex for use as a diagnostic reagent in the microtiter technique, comprising a colored latex prepared by dyeing polyacrolein with a dye.

4.      A latex according to claim 1, 2 or 3, wherein the dye is an amino group-containing basic dye or acid dye.

5.      A latex according to claim 1, 2 or 3, wherein the dye is a hydrophobic fluorescent dye or disperse dye.

6.      A latex according to claim 1, 2 or 3, wherein the particle diameter of the polyacrolein particles is 0.1 to 5 µm.

7.      A latex according to claim 1, 2 or 3, wherein the dispersant of the latex is water.

# INTERNATIONAL SEARCH REPORT

0305536

International Application No PCT/JP88/00213

## I. CLASSIFICATION OF SUBJECT MATTER (if several classification symbols apply, indicate all) ³

According to International Patent Classification (IPC) or to both National Classification and IPC

Int.Cl⁴ $C08L29/12$, $G01N33/545$

## II. FIELDS SEARCHED

| Minimum Documentation Searched ⁴ | |
|---|---|
| Classification System | Classification Symbols |
| IPC | $C08L29/00$, $G01N33/543-33/556$ |

| Documentation Searched other than Minimum Documentation<br>to the Extent that such Documents are Included in the Fields Searched ⁵ | |
|---|---|
| Jitsuyo Shinan Koho | 1970 – 1987 |
| Kokai Jitsuyo Shinan Koho | 1970 – 1987 |

## III. DOCUMENTS CONSIDERED TO BE RELEVANT ¹⁴

| Category * | Citation of Document, ¹⁶ with indication, where appropriate, of the relevant passages ¹⁷ | Relevant to Claim No. ¹⁸ |
|---|---|---|
| A | US, A, 4552812 (Yeda Research and Development Company, Ltd.) 12 November 1985 (12. 11. 85) & DE, A, 3224484 | 1-7 |
| A | JP, A, 59-51938 (Yeda Research and Development Company, Ltd.) 26 March 1984 (26. 03. 84) & EP, A, 87786 & DE, A, 3372211 | 1-7 |

* Special categories of cited documents: ¹⁵

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search ² | Date of Mailing of this International Search Report ² |
|---|---|
| April 1, 1988 (01. 04. 88) | April 18, 1988 (18. 04. 88) |
| International Searching Authority ¹ | Signature of Authorized Officer ²⁰ |
| Japanese Patent Office | |

Form PCT/ISA/210 (second sheet) (October 1977)